(19) Europäisches Patentamt
European Patent Office
Office européen des brevets

(11) Numéro de publication: 0 231 234 B1

(12) FASCICULE DE BREVET EUROPEEN

(45) Date de publication du fascicule du brevet: 04.07.90

(51) Int. Cl.$^5$: C 12 P 17/18

(21) Numéro de dépôt: 86904226.7

(22) Date de dépôt: 22.07.86

(86) Numéro de dépôt international: PCT/FR86/00260

(87) Numéro de publication internationale: WO 87/00552 29.01.87 Gazette 87/03

# (54) PROCEDE D'HYDROXYLATION PAR VOIE MICROBIOLOGIQUE DE LA QUININE, DE LA QUINIDINE, ET DE DERIVES.

(30) Priorité: 23.07.85 FR 8511221

(43) Date de publication de la demande: 12.08.87 Bulletin 87/33

(45) Mention de la délivrance du brevet: 04.07.90 Bulletin 90/27

(84) Etats contractants désignés: AT BE CH DE FR GB IT LI LU NL SE

(56) Documents cités:
EP-A-0 066 489

Journal of Natural Products, vol. 47, no. 5, septembre-octobre 1984; F.M. Eckenrode:" Microbiological transformation of quinidine", pp. 882-884

Le dossier contient des informations techniques présentées postérieurement au dépôt de la demande et ne figurant pas dans le présent fascicule.

(73) Titulaire: LABORATOIRE NATIVELLE (Société Anonyme)
70, rue du Gouverneur Général Eboué
F-92130 Issy les Moulineaux (FR)

(72) Inventeur: JARREAU, François-Xavier
5, rue Louis Hervé
F-78000 Versailles (FR)
Inventeur: AZERAD, Robert
13bis, rue Berthelot
F-91130 Ris Orangis (FR)
Inventeur: OGERAU, Thierry
27, avenue Victor Hugo
F-78960 Voisins le Bretonneux (FR)

(74) Mandataire: L'Helgoualch, Jean
Cabinet Yvette SUEUR 35, rue de la Frette
F-95240 CORMEILLES EN PARISIS (FR)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 231 234 B1

L'invention concerne un procédé d'hydroxylation d'alcaloïdes du quinquina, et plus particulièrement un nouveau procédé d'hydroxylation de la quinine, de la quinidine, et de dérivés, par voie microbiologique.

L'hydroxylation microsomale hépatique constitue souvent l'une des premières étapes du métabolisme d'un composé chimique étranger introduit dans l'alimentation ou la circulation sanguine des mammifères. Ces hydroxylations, suivies d'autres réactions, comme la O-méthylation ou la conjugaison, sont destinées à rendre ces substances plus hydrosolubles, donc à accélérer leur élimination. Cependant dans certains cas, les dérivés hydroxylés se trouvent être plus actifs, ou plus toxiques, que la molécule originale, ce qui justifie l'intérêt de leur identification, et par suite de leur préparation pour l'étude de leur activité.

Les méthodes de synthèse organique présentent des facilités évidentes pour les métabolites de substances simples, mais la préparation chimique de métabolites de molécules plus complexes (et optiquement actives) peut nécessiter une quantité d'efforts et de temps considérable.

Les hydroxylations par voie microbiologique ont déjà été considérablement développées depuis de nombreuses années, dans le domaine des stéroides, où elles ont permis l'obtention, à l'échelle industrielle, de produits hydroxylés régio- et stéréosélectivement, résultant de l'oxydation spécifique d'atomes de carbone non fonctionalisés. De telles molécules auraient demandé des efforts synthétiques considérables si des méthodes purement chimiques avaient dû être employées. Cette méthode a depuis été appliquée à d'autres familles de molécules et constitue, dans certains cas, un mode privilégié d'obtention de composés nouveaux, transposable à grande échelle, en utilisant les techniques et les équipements classiques des fermentations. De plus, il apparaît que les hydroxylations microbiennes conduisent pour chaque espèce à un éventail de métabolites souvent beaucoup plus restreint que celui des systèmes de mammifères, ce qui élimine certains problèmes de séparation; il suffit alors de jouer sur la multiplicité des souches pour reconstituer la variété éventuelle des métabolites hépatiques.

Toutefois, dans le domaine des alcaloïdes, assez peu de réactions de ce type ont été décrites, et elles portent pour la plupart sur la partie non azotée de la molécule, aromatique ou stéroïdique. Il est pourtant souhaitable et intéressant de pouvoir effectuer dans de bonnes conditions l'hydroxylation de noyaux azotés d'alcaloïdes dont les produits ont déjà été reconnus comme des métabolites actifs. En particulier, le brevet français 2.506.312 décrit des dérivés hydroxylés de la quinidine, tels que l'hydroxy-3 dihydro-10,11 quinidine et ses épimères 3R et 3S, utilisables en thérapeutique pour le traitement des arythmies cardiaques. On sait par ailleurs que la quinine, dont les propriétés pharmacologiques sont connues, et la quinidine sont deux isomères se différenciant par leurs configurations au niveau des atomes de carbone en positions 8 et 9.

La préparation de ces composés actifs, optiquement purs, par une méthode microbiologique transposable à l'échelle industrielle représente donc un objectif d'un intérêt évident, par rapport aux hydroxylations de type chimique qui conduisent, avec un rendement médiocre, à un mélange diastéréoisomérique. L'hydroxylation de la quinidine par certains micro-organismes est décrite par Eckenrode *J. Nat. Prod.* vol. 47, 5 (1984) pp. 882—884.

La présente invention a donc pour objet un nouveau procédé d'hydroxylation microbiologique de la quinine, la quinidine, et leurs dérivés, en particulier leurs dérivés hydrogénés, de manière régio- et stérospécifique, pour obtenir des composés optiquement purs.

L'invention a également pour objet un procédé d'hydroxylation microbiologique de la quinine, la quinidine, et leurs dérivés, susceptible d'être mis en oeuvre industriellement et de procurer des composés optiquement purs avec un rendement satisfaisant au moyen d'un appareillage de type conventionnel.

Le procédé d'hydroxylation microbiologique régiospécifique et stéréospécifique suivant l'invention, permet d'hydroxyler en position 3S la quinine, la quinidine et leurs dérivés représentés par la formule (I):

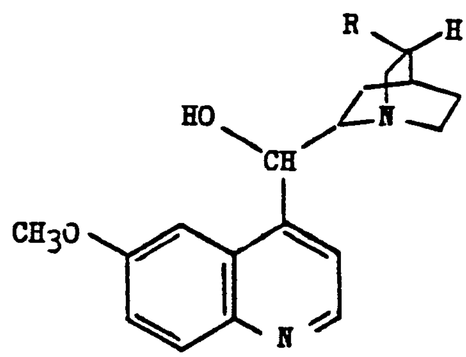

I

dans laquelle R représente un groupe éthyle ou un groupe vinyle pour former les dérivés 3S-hydroxylés correspondants, de formule (II):

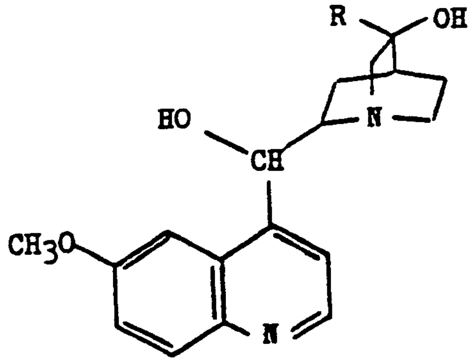

II

dans laquelle R a la même signification que ci-dessus, en faisant agir sur les composés de formule (I) un microorganisme du type champignon inférieur choisi dans le groupe constitué par: Cunninghamella echinulata, Mucor circinelloïdes, Mucor plumbeus, ou du type bactérien tel que Streptomyces rimosus avec la réserve que C. echinulata n'est pas appliqué à l'hydroxylation de la quinidine.

Dans la formule (I) ci-dessus, R représente un groupe vinyle dans le cas de la quinine et de la quinidine, qui se différencient par leurs configurations au niveau des atomes de carbone en positions 8 et 9, et R représente un groupe éthyle dans le cas des dérivés hydrogénés, c'est-à-dire la dihydroquinine et la dihydroquinidine. La même observation vaut pour les dérivés hydroxylés de formule (II).

On utilise de préférence les souches identifiées ci-après: Cunninghamella echinulata (NRRL 3655, MMP 2203), Mucor circinelloides (CBS 108—16), Mucor plumbeus (MMP 430, CBS 111—07), Streptomyces rimosus (NRRL 2234), et le procédé consiste à faire agir l'un des microorganismes ci-dessus sur le composé à hydroxyler.

Les références ci-dessus ont les significations connues, c'est-à-dire, ATCC: American Type Culture Collection, Rockville, Md. (USA); CBS: Centraal Bureau voor shimmelcultures, Baarn (Pays-Bas); MMP: Mycothèque du Museum d'Histoire Naturelle, Paris (France).

Suivant le procédé d'hydroxylation de l'invention, il est préférable de faire croître le microorganisme dans un milieu de culture contenant des sources assimilables de carbone, d'azote et de sels minéraux, dans des conditions de fermentation aérobie, en culture immergée. Le composé à hydroxyler est ajouté, de préférence stérilement, au milieu, on laisse incuber jusqu'à obtenir une quantité suffisante de produit hydroxylé, et on extrait par les techniques usuelles. L'incubation peut s'effectuer dans un fermenteur de type usuel, à une température comprise entre 20 et 30°C environ, le pH du milieu étant voisin de 7. On peut le cas échéant utiliser un milieu dont le pH est compris entre 6 et 8 environ, sans modifier sensiblement les résultats.

Le procédé utilisé consiste à faire pousser les microorganismes dans le milieu de culture adéquat afin d'obtenir une biomasse bien développée; ce milieu peut contenir à côté d'une source de carbone assimilable (glucose, saccharose, amidon, mannitol, purs ou en mélange avec d'autres carbohydrates) à une concentration de 1 à 10%, une source d'azote organique (peptones, asparagine, hydrolysats protéiques) ou minéral (sels d'ammonium, nitrates), et les sels minéraux nécessaires ainsi que des substances vitaminiques (telles que celles présentes dans le "corn steep", l'extrait de levure, . . .); la température peut varier de 20 à 30°C environ selon les souches, et la culture est effectuée en milieu agité. Lorsque la croissance est jugée satisfaisante et en général lorsque le carbohydrate substrat est épuisé, c'est-à-dire au bout de 2 à 5 jours environ, on ajoute stérilement le composé à hydroxyler, solide ou en solution dans un petit volume de solvant organique miscible à l'eau (par exemple l'éthanol), à une concentration finale pouvant aller de 0,2 à plusieurs g./litre suivant la molécule ou la souche. L'incubation, qui peut s'accompagner d'une faible croissance résiduelle, est poursuivie, en agitant vigoureusement pour aérer le mélange, aux mêmes températures, pendant un temps de 2 à 40 jours.

Suivant une variante du procédé de l'invention, l'incubation du composé à hydroxyler est faite avec des cellules préalablement lavées et remises en suspension dans un milieu aqueux tamponné ne contenant pas de source de carbone ou d'azote. L'hydroxylation est alors effectuée après avoir fait croître le microorganisme et après avoir récolté le mycélium, en absence de croissance.

L'extraction des produits de l'incubation ainsi que celle du substrat résiduel est effectuée à l'aide d'un solvant organique non miscible à l'eau (chlorure de méthylène, chloroforme, acétate d'éthyle, etc) après avoir éliminé le mycélium par filtration et saturé le filtrat en sels (NaCl, $Na_2SO_4$).

La détection et l'identification des produits d'hydroxylation peut être effectuée par chromatographie sur couche mince de gel de silice ou par chromatographie liquide haute pression sur phase inverse. La séparation du produit hydroxylé du substrat de départ éventuellement restant et sa purification sont effectuées par les techniques classiques de cristallisation ou de chromatographie sur adsorbants minéraux.

Les produits d'hydroxylation purifiés ont été identifiés par comparaison aux produits (3S)-hydroxy authentiques (préparés par voie chimique ou décrits dans la littérature) dans divers systèmes chromatographiques, ainsi que par les techniques spectroscopiques (UV,$^1$H- ou $^{13}$C-RMN) et polarimétriques usuelles.

Les résultats obtenus sont rapportés dans les exemples suivants où plusieurs souches de microorganismes capables de réaliser ces hydroxylations d'une manière régio- et stéréosélective, à partir de plusieurs des substrats envisagés, avec un taux de conversion variable, ont été caractérisées. Outre cet avantage essentiel de la sélectivité de l'hydroxylation, un autre avantage est l'absence de formation d'autres métabolites en quantités significatives, ce qui simplifie la purification et permet le recyclage du substrat éventuellement non métabolisé.

Les exemples suivants illustrent l'invention sans en limiter la portée.

Exemple 1

Hydroxylation de la quinidine par M.plumbeus

A 100 ml de milieu A décrit ci-après, ajusté à pH 7, dans un erlenmeyer de 250 ml, sont ajoutés quelques gouttes d'une suspension de spores de M.plumbeus MMP 430. On incube 72 heures à 27°C dans un agitateur rotatif (327 trs/mn). On ajoute alors stérilement à la culture une solution de 50 mg de quinidine dans 1 ml d'éthanol et on continue à agiter 14 jours dans les mêmes conditions. La culture est filtrée sur célite, le filtrat saturé par NaCl amené à pH 10—12 par NaOH 2N puis extrait 6 fois au chlorure de méthylène. Les fractions organiques séchées puis évaporées sont chromatographiées sur couche préparative de gel de silice en utilisant le solvant $CHCl_2$—MeOH—$NH_4OH$ (85:14:1). La bande de (3S)-3-hydroxy quinidine, repérée par sa fluorescence à 254 nm et sa migration inférieure à celle de la guinidine, est grattée et éluée par le méthanol. On obtient 1 mg de (3S)-3-hydroxy quinidine identique sous tous rapports à un échantillon authentique. La guinidine non réagie est récupérée de la même manière presque quantitativement (42 mg).

Milieu A:

| | |
|---|---|
| corn steep | 10 g |
| $MgSO_4,7H_2O$ | 0,5 g |
| KCl | 0,5 g |
| $NaNO_3$ | 2 g |
| $FeSO_4,7_2O$ | 0,02 g |
| D-glucose | 30 g |
| $KH_2PO_4$ | 1 g |
| $K_2HPO_4$ | 2 g |

eau distillée, pour compléter à 1 litre.

Exemple 2

Hydroxylation de la dihydroquinidine par M.plumbeus

La même incubation, effectuée sue une culture analogue de M.plumbeus CBS 111—07 avec 1 g/litre en concentration finale de dihydroquinidine, fournit après 20 jours 6 mg de (3S)-3-hydroxy dihydroquinidine identique à un échantillon authentique et 80 mg de dihydroquinidine récupérée.

Exemple 3

Hydroxylation de la dihydroquinidine par S.rimosus

A 100 ml de milieu B décrit ci-après, ajusté à pH 7, dans un erlenmeyer de 250 ml, sont ajoutés quelques ml d'une préculture de S.rimosus NRRL 2234. On cultive 72 heures à 27°C; on ajoute 100 mg de dihydroquinidine en solution dans 1 ml d'éthanol et on continue à agiter pendant 25 jours dans les mêmes conditions. On obtient de la même manière 4 mg de (3S)-3-hydroxy dihydroquinidine et 85 mg de dihydroquinidine récupérée.

Milieu B:

| | |
|---|---|
| Bacto Casamino acids Difco | 5 g |
| extrait de levure | 3 g |
| D-glucose | 30 g |

eau distillée, pour compléter à 1 litre.

Exemple 4

Hydroxylation de la quinine par C.echinulata

La même incubation, effectuée sur une culture analogue (milieu A) de C.echinulata MMP 2203, avec 1 g/litre en concentration finale de quinine, fournit après 28 jours 10,5 mg de (3S)-3-hydroxy quinine identique à un échantillon authentique et 74 mg de quinine récupérée. On constate également la formation de deux autres produits (fluorescents à 254 nm) en faible quantité.

Exemple 5

Hydroxylation de la dihydroquinine par M.circinelloides

La même incubation, effectuée sur une culture analogue (milieu A) de M.circinelloides CBS 108—16, avec 1 g/litre en concentration finale de dihydroquinine, fournit après 36 jours 17 mg de (3S)-3-hydroxy dihydroquinine identique à un échantillon authentique et 70 mg de dihydroquinine récupérée.

Exemple 6

Hydroxylation de la dihydroquinidine par M.plumbeus

Deux cultures de 100 ml de M.plumbeus MMP430 sur le même milieu A que dans l'exemple 1, sont réunis, centrifugées, et le mycélium, lavé stérilement avec un tampon phosphate contenant 2 g de $K_2HPO_4$ et 1 g de $KH_2PO_4$ par litre d'eau distillée, est repris dans 100 ml du même tampon et incubé avec 0,5 g/litre en concentration finale de dihydroquinidine.

Après 5 jours d'agitation à 27°C, on obtient 6,5 mg de (3S)-3-hydroxy dihydroquinidine, et 40 mg de dihydroquinidine récupérée, par les techniques usuelles.

**Revendications**

1. Procédé d'hydroxylation microbiologique régiospécifique et stéréospécifique, en position 3S, de la quinine, la quinidine et leur dérivés, caractérisé en ce qu'on fait agir un microorganisme du type champignon inférieur choisi dans le groupe constitué par Cunninghamella echinulata, Mucor circinelloides, Mucor plumbeus, ou du type bactérien tel que Streptomyces rimosus, sur le composé à hydroxyler avec la réserve que C. echinulata n'est pas appliqué à l'hydroxylation de la quinidine.

2. Procédé d'hydroxylation selon la revendication 1, caractérisé en ce qu'on fait croître le microorganisme dans un milieu de culture contenant des sources assimilables de carbone, d'azote et de sels minéraux, dans des conditions de fermentation aérobie, en culture immergée.

3. Procédé selon l'une quelconque des revendications 1 et 2, caractérisé en ce que le composé à hydroxyler est ajouté directement au milieu de culture lorsque la croissance du microorganisme est suffisante.

4. Procédé selon l'une quelconque des revendications 1 et 2, caractérisé en ce que l'incubation du composé à hydroxyler est faite avec des cellules préalablement lavées et remises en suspension dans un milieu en contenant pas de source de carbone ou d'azote.

5. Procédé d'hydroxylation selon la revendication 2, caractérisé en ce que l'incubation s'effectue à une température comprise entre 20 et 30°C.

6. Procédé d'hydroxylation selon l'une quelconque des revendications 1 et 2, caractérisé en ce que le pH du milieu de culture est compris entre 6 et 8 environ.

**Patentansprüche**

1. Verfahren zur regiospezifischen und stereospezifischen mikrobiologischen Hydroxylierung von Chinin, Chinidin und ihren Derivaten in der 3S-Position, dadurch gekennzeichnet, daß man einem Mikroorganismus vom niederen Champignon-Typ, ausgewählt aus der Gruppe, die besteht aus Cunninghamella echinulata, Mucor circinelloides und Mucor plumbeus, oder vom Bakterien-Typ, wie Streptomyces rimosus, auf die zu hydroxylierende Verbindung einwirken läßt, jedoch mit der Ausnahme, daß C. echinulata nicht zur Hydroxylierung von Chinidin verwendet wird.

2. Verfahren nach Anspruch 1, dadurch gekennzeichnet, daß man den Mikroorganismus in einem Kulturmedium, das assimilierbare Kohlenstoff-, Stickstoff- und Mineralsalz-Quellen enthält, unter aeroben Fermentationsbedingungen in einer Immersionskultur wachsen läßt.

3. Verfahren nach Anspruch 1 oder 2, dadurch gekennzeichnet, daß man die zu hydroxylierende Verbindung direkt dem Kulturmilieu zusetzt, wenn das Wachstum der Mikroorganismen ausreichend ist.

4. Verfahren nach Anspruch 1 oder 2, dadurch gekennzeichnet, daß man die Inkubation der zu hydroxylierenden Verbindung mit vorher gewaschenen und in einem Milieu, das keine Kohlenstoff- oder Stickstoffquelle enthält, wieder suspendierten Zellen durchführt.

5. Verfahren nach Anspruch 2, dadurch gekennzeichnet, daß man die Inkubation bei einer Temperatur zwischen 20 und 30°C durchführt.

6. Verfahren nach Anspruch 1 oder 2, dadurch gekennzeichnet, daß man den pH-Wert des Kulturmediums zwischen etwa 6 und etwa 8 hält.

## Claims

1. Process for the regiospecific and stereospecific hydroxylation of quinine, quinidine and their derivatives, in position 3S, characterized in that a microorganism of the lower fungus type selected from the group constituted by Cunninghamella echinulata, Mucor circinelloides, Mucor plumbeus, or the bacterial type such as Streptomyces rimosus, is reacted on the compound to be hydroxylated, with the proviso that C. echinulata is not applied to the hydroxylation of quinidine.

2. Hydroxylation process according to claim 1, characterized in that the microorganism is grown in a culture medium containing assimilable sources of carbon, nitrogen, and mineral salts, under conditions of aerobic fermentation in immersed culture.

3. Process according to any of claim 1 or 2, characterized in that the compound to be hydroxylated is added directly to the culture medium when the growth of the microorganism is sufficient.

4. Process according to any of claim 1 or 2, characterized in that the incubation of the compound to be hydroxylated is carried out directly with previously washed cells, which are resuspended in a medium which does not contain any source of carbon or nitrogen.

5. Hydroxylation process according to claim 2, characterized in that the incubation is carried out in the temperature range 20 to 30°C.

6. Process according to any of claim 1 or 2, characterized in that the pH of the culture medium is in the range between 6 and 8, approximately.